# EUROPÄISCHE PATENTANMELDUNG

(11) **EP 2 166 081 A2**
(43) Veröffentlichungstag der Anmeldung: **24.03.2010**
(21) Anmeldenummer: 08019718.9
(22) Anmeldetag: 06.11.2008
(51) Int. Cl.: C12M 1/107

(54) **Biogasanlage mit einem Fermenterbehälter**

(30) Priorität: 18.09.2008 DE 102008047828
(71) Anmelder: Chowdhury, Rajib Pal, 83512 Wasserburg (DE)
(72) Erfinder: Chowdhury, Rajib, Pal, 83512 Wasserburg (DE); Bürger, Adam, 83527 Haag/Obb. (DE)

(57) **Zusammenfassung**

Die Erfindung betrifft eine Biogasanlage (1) mit einem Fermenterbehälter (2). Erfindungsgemäß weist der Fermenterbehälter (2) mit einer rechteckigen Grundform eine Bodenwand (8), zwei gegenüberliegende Längswände (9, 10), zwei gegenüberliegende Stirnwände (11, 12) sowie eine obere Abdeckung (17) auf. Der Fermenterbehälter (2) besteht zur Ausbildung seiner Wände aus einem quaderförmigen Stützkäfig (23) mit einer Gitterstruktur aus miteinander verbundenen, wandseitigen Vertikalstützen (24), Längsträgern (27), Querträgern (28) und gegebenenfalls Zugelementen (30, 32) und/oder Aussteifungselementen (31), wobei der Stützkäfig (23) zumindest zur Ausbildung der Längswände (9, 10) und Stirnwände (11, 12) mit Plattenelementen beplankt ist. Die Bodenwand (8), die Längswände (9, 10) und die Stirnwände (11, 12) sind mit einer fermenterbehälterinneren, durchgehenden und dichten Innenfolie (39) ausgekleidet und abgedeckt.

## Beschreibung

Die Erfindung betrifft eine Biogasanlage mit einem Fermenterbehälter nach dem Oberbegriff des Anspruchs 1.

Biogasanlagen bestehen allgemein aus wenigstens einem Fermenterbehälter und umfassen weiter eine Beschickungseinrichtung für zu vergärende Biomasse, eine Umwälzeinrichtung für ein Substrat im Fermenterbehälter sowie eine Heizeinrichtung und einen Gasspeicher. Meist ist dem Gasspeicher ein Blockheizkraftwerk nachgeordnet, wo in einem Gasmotor das Biogas verbrannt und in einem angeschlossenen Generator elektrische Energie erzeugt wird.

Fermenterbehälter für solche Biogasanlagen werden in unterschiedlichen Ausführungsformen gebaut. Meist werden große kreisringförmige Ringbehälter aus Beton oder Stahl mit vertikaler Achse als Fermenterbehälter verwendet, die mit einer starren Abdeckung oder einem Zeltdach gegebenenfalls als Gasspeicher abgedeckt sind. Solche Anlagen werden regelmäßig als individuell geplante, relativ große Anlagen hergestellt. Entsprechend hoch ist der Planungs- und Herstellaufwand, da insbesondere ein Großteil der Montage weitgehend individuell auf der Baustelle erfolgen muss.

Während bisher eher der Trend zu immer größeren Biogasanlagen im MW-Bereich mit dafür geeigneten Entwicklungen vorgelegen hat, ergeben sich durch das EEG 2009 (Energieeinspeisungsgesetz 2009) für kleinere landwirtschaftliche Biogasanlagen mit einer elektrischen Leistung kleiner als 150 kW wirtschaftliche interessante Einspeisevergütungen. Solche Biogasanlagengrößen sind insbesondere für mittelgroße landwirtschaftliche Betriebe mit etwa 100 bis 200 Großvieheinheiten geeignet, womit einerseits anfallende Gülle veredelt werden kann und andererseits elektrische Energie zur Verfügung gestellt wird, die gewinnbringend in ein Netz eingespeist werden kann. Die entstehende Wärme kann ebenfalls im eigenen Betrieb genutzt oder verkauft werden.

Aufgabe der Erfindung ist es, einen Fermenterbehälter für solche relativ kleinen Biogasanlagen vorzuschlagen, der besonders einfach aufgebaut, kostengünstig herstellbar und schnell montierbar ist.

Diese Aufgabe wird mit den Merkmalen des Anspruchs 1 gelöst.

Gemäß Anspruch 1 weist der Fermenterbehälter mit einer rechteckigen Grundform eine Bodenwand, zwei gegenüberliegende Längswände, zwei gegenüberliegende Stirnwände sowie eine obere Abdeckung auf. Der Fermenterbehälter besteht zur Ausbildung seiner Wände aus einem quaderförmigen Stützkäfig mit einer Gitterstruktur aus miteinander verbundenen, wandseitigen Vertikalstützen, Längsträgern, Querträgern und gegebenenfalls Zugelementen und/oder Aussteifungselementen. Zudem ist der Stützkäfig zumindest zur Ausbildung der Längswände und Stirnwände mit Plattenelementen beplankt. Die Bodenwand, die Längswände und die Stirnwände sind mit einer fermenterbehälterinneren, durchgehenden und dichten Innenfolie ausgekleidet und abgedeckt.

Ein so hergestellter Fermenterbehälter ist besonders einfach und kostengünstig herzustellen, wobei die einzelenen Bauelemente standardisierte Elemente eines werkseitig herstellbaren Fermenterbehälter-Montagebausatzes sein können, wodurch insbesondere Montagezeiten und Montagekosten auf der Baustelle erheblich reduziert sind. Die einzelnen Bauelemente können zudem in Abmessungen hergestellt werden, die einen einfachen Straßentransport erlauben, so dass es wirtschaftlich interessant sein kann solche Biogasanlagen auch in weiterer Entfernung vom Herstellort des Bausatzes beispielsweise im Ausland anzuliefern und aufzustellen, wodurch sich neue Geschäftsbereiche ergeben.

Insbesondere können damit Kompaktanlagen als Biogas-Kleinanlagen nach dem neuen EEG 2009 hergestellt und betrieben werden.

### Weiter ergeben sich finanztechnische Vorteile:

Wegen der vergleichsweise extrem kurzen Bauzeit braucht ein Kunde regelmäßig keine Zwischenfinanzierung durch eine Bank. Da eine Biogasanlage mit einem aus einem Fermenter-Montagebausatz hergestellten Fermenterbehälter problemlos montierbar aber auch gegebenenfalls wieder demontierbar ist, sind Geschäftsmodelle möglich, in denen solche Biogasanlagen Betreibern über Leasing-Gesellschaften oder durch Investoren zur Benutzung zur Verfügung gestellt werden können. Jedenfalls kann eine so hergestellte Biogasanlage wegen seiner gegebenenfalls einfachen Demontage als Gebrauchtanlage auch wieder verkauft und an einem anderen Ort aufgestellt werden.

Ein weiterer Vorteil ist der durch die rechteckige Bauweise geringe Platzbedarf bei Verwendung einfacher rechteckiger, planer Plattenelemente im Vergleich zu zylindrischen Fermenterbehältern.

Mit Anspruch 2 werden Merkmale und Aufbauten beansprucht, die den Bausatzcharakter für eine kompakte Kleinbiogasanlage weiter verdeutlichen und zu einer Reduzierung der Kosten bei einer Effizienzsteigerung beitragen können. Dazu bilden die Gitterstrukturelemente des Stützkäfigs und die Plattenelemente der Beplankung im nicht montierten Zustand einen Fermenterbehälter-Montagebausatz, aus dem mit lösbaren Verbindungen Fermenterbehälter in gestuften, standardisierten Größen herstellbar sind. Vorzugsweise liegen diese Größen in einem Größenbereich von 24m Länge, 12m Breite und 4m Höhe, wobei diese Größe erfahrungsgemäß für eine elektrische Leistung von ca. 150kW der Biogasanlage geeignet ist. Zudem sind aus dem Montagesatz mit entsprechend weniger Bauteilen dagegen abgestufte Größen für ca. 100kW, 70kW und 35kW herstellbar. Mit diesen abgestuften Standardtypen sind in einer Art von Serienherstellung weitere erhebliche Kostenvorteile zu erzielen.

Zu einer Steigerung der Gesamteffektivität sollen weiter der Fermenterbehälter sowie gegebenenfalls ein vorgefertigt zum Fermenterbehälter-Bausatz gehörender BHKW- und Technikcontainer und gegebenenfalls weitere zur Biogasanlage gehörende und neben dem Fermenterbehälter angeordnete Vorrichtungen mit einem Pultdach als Photovoltaikdach überdacht werden. Zum Voltaikdach gehörende Elektrogeräte können zudem einfach, platzsparend und kostengünstig mit im Technikcontainer aufgenommen sein.

Eine konstruktiv einfache Ausbildung des Stützkäfigs mit einer geeigneten Stützfunktion wird mit den Merkmalen des Anspruchs 3 angegeben. Dazu sind die Gitterstrukturelemente des Stützkäfigs gebildet aus bodenseitigen unteren Längsträgern und unteren Querträgern, aus denen ein unterer Rechteckrahmen hergestellt ist, aus Vertikalstützen, die am unteren Rechteckrahmen gegeneinander beabstandet jeweils mit seinem unteren Endbereich befestigt sind und aus oberen Längsträgern und oberen Querträgern, aus denen ein oberer Rechteckrahmen hergestellt ist, der mit jeweils einem oberen

Endbereich der Vertikalstützen verbunden ist.

Entsprechend der statischen Erfordernisse wird nach Anspruch 4 im Bodenwandfeld des unteren Rechteckrahmens ein Zugbandnetz angebracht. Das Zugbandnetz ist vorzugsweise aus trägerparallelen Metallflachbändern gebildet, die die gegenüberliegenden Längsträger und Querträger verbinden und bevorzugt im Bereich der Befestigungsstellen der Vertikalstützen angeschlossen sind.

Entsprechend sind nach Anspruch 5 auch in der oberen Horizontalebene des oberen Rechteckrahmens zwischen gegenüberliegenden Längsträgern und/oder zwischen gegenüberliegenden Querträgern trägerparallele Aussteifungselemente, vorzugsweise Aussteifungsrohre und/oder schräg zu den Trägern verlaufende und sich überkreuzende Zug- und/oder Druckelemente, vorzugsweise Edelstahlseile oder Kreuzstreben angebracht. Zudem können für eine weitere Aussteifung und Abstützung von auf die Seitenwände gerichtete Schub-, Zug- und Ausbeulkräfte weitere Versteifungen dadurch vorgenommen werden, dass parallel und beabstandet zum unteren Rechteckrahmen weitere Längsträger und Querträger mit den Vertikalstützen verbunden sind, die vorzugsweise wieder einen geschlossenen Rechteckrahmen bilden und/oder dass in den Seitenwandfeldern insbesondere zwischen den Vertikalstützen Versteifungselemente und Zugelemente, vorzugsweise Metallstäbe in Kreuzanordnungen angebracht sind.

Gemäß Anspruch 6 kann der Stützkäfig ganz oder teilweise aus Metall hergestellt sein, wobei dann die Vertikalstützen, die Längsträger und die Querträger als Stahl-Doppel-T-Träger und/oder als Stahlwinkelträger ausgeführt sind. Insbesondere bei relativ kleinen Biogasanlagen kann der Stützkäfig in einer besonders umweltfreundlichen Konstruktion auch aus Holz gegebenenfalls mit einer Holzbeplankung ausgeführt sein. Für eine einfache Montage und gegebenenfalls eine Demontage werden die einzelnen Elemente des Stützkäfigs miteinander verschraubt.

Die Ausführung der Bodenwand des Fermenterbehälters ist gemäß Anspruch 7 unkritisch, wobei in einfachster Weise nur der planbefestigte Erdboden verwendet wird. Es kann auch eine unbewehrte Bodenplatte aus Magerbeton hergestellt werden, die sehr einfach und kostengünstig herstellbar und auch ebenso einfach bei Bedarf wieder entfernbar ist. Auf dem Erdboden oder der Magerbetonplatte wird eine Wärmedämmschicht aus Wärmedämmplatten aufgelegt, auf der dann der Bodenbereich der Innenfolie aufliegt. Ein bodenseitiges Zugbandnetz kann bei diesem Aufbau abgedeckt oder eingebettet werden, worauf dann auf dieser Abdeckschicht die Wärmedämmschicht und die Innenfolie aufliegt.

Die Beplankung, die aufgrund der Stützfunktion des Stützkäfigs nur relativ geringe Kräfte aufnehmen muss, kann daher aus unterschiedlichen Materialien und unterschiedlichem Lagenaufbau hergestellt sein. Bevorzugt werden jedoch gemäß Anspruch 8 Plattenelemente für die Beplankung in der Art von wasserfesten Sandwichpaneelen mit einem integrierten Dämmmaterialkern verwendet, welche marktüblich bezogen werden können und durch die integrierte Wärmedämmung hinsichtlich der Funktion und der einfachen Montage sehr gut geeignet sind. Andererseits können jedoch für eine Beplankung auch Stahlplatten mit aufgebrachter Wärmedämmschicht und Verkleidung oder auch Kunststoffplatten oder auch Holzfaserplatten oder Holzbretter mit entsprechend aufgebrachter Wärmedämmschicht und Verkleidung eingesetzt werden.

Nach Anspruch 9 können Plattenelemente als Rechteckplatten mit einer Länge entsprechend der Fermenterbehälterhöhe vertikal ausgerichtet zur Herstellung der Beplankung von innen her an den Stützkäfigseitenwänden angebracht werden, wodurch eine gute Abstützfunktion bei schneller Montage erreicht wird. Alternativ dazu können entsprechende Rechteckplatten mit einer Länge entsprechend der Breite eines Seitenwandfeldes horizontal gerichtet zwischen Vertikalstützen verbaut werden.

In einer besonders bevorzugten Weiterbildung nach Anspruch 10 weist der Fermenterbehälter eine jeweils zu den Stirnwänden beabstandete StrömungsLängsmittenwand auf, die vorzugsweise als Montagemodul zum Fermenterbehälter-Montagebausatz gehört und vorgefertigt ist. Weiter ist im Fermenterbehälter ein Rührwerk als an sich bekanntes Tauchrührwerk oder als Stabrührwerk zum Aufbau einer ringförmigen Substratströmung um die Strömungs-Längsmittenwand herum vorgesehen. Zur Ausbildung einer geeigneten Strömung ist bevorzugt der Abstand der StrömungsLängsmittenwand beidseitig zu den Stirnwänden gleich und entspricht etwa der Breite des Fermenterbehälters. Mit einer solchen Ausbildung wird ein effektives Rühr- und Mischergebnis mit relativ wenig Energieaufwand für das Substrat im Fermenterbehälter erzielt.

Es besteht auch die Möglichkeit zusätzlich Leitelemente zur noch besseren Durchmischung der Substrate einzubauen.

In einer weiteren vorteilhaften Zusatzfunktion werden nach Anspruch 11 auf der Strömungslängsmittenwand und/oder in dieser integriert Heizelemente für die Fermenterheizung angebracht.

In einer dazu gut geeigneten konkreten Ausführungsform nach Anspruch 12 sind an der Strömungs-Längsmittenwand beidseitig vorzugsweise parallel und im unteren Dritten verlaufende Heizungsrohre angeordnet, die aus Kunststoffmaterial oder Edelstahl bestehen können. Die StrömungsLängsmittenwand kann dabei aus einem festen Material hergestellt sein oder gegebenenfalls aus einem an einem Gestell befestigten flexiblen Gewebe bestehen.

Aus geeigneten Materialen für die Innenfolie werden mit Anspruch 13 eine gewebeverstärkte PVC-Folie und/oder eine FPP-Dichtungsbahn angegeben.

Weitere Bauelemente, insbesondere solche, die der Atmosphäre des Fermenterinnenbereichs ausgesetzt sind, werden zum Schutz vor Korrosion und Verschleiß vorzugsweise aus V4A-Stahl hergestellt oder mit Epoxidharz beschichtet.

Die obere Abdeckung des Fermenterbehälters kann gegebenenfalls als flache Decke ausgeführt sein, wobei auch hier gegebenenfalls Plattenelemente verwendbar sind. Bevorzugt wird jedoch die obere Abdeckung des Fermenterbehälters nach Anspruch 14 als Zeltdach ausgeführt, insbesondere als doppelwandiges Zeltdach mit zwei Dachmembranen und integriertem Gasspeicher. Zum Schutz der inneren Dachmembran ist zudem ein in der Höhe der Längswände und Stirnwände bei entsprechender Höhe der Strömungslängsmittenwand längs- und/oder querverlaufendes Gurtnetz aufgespannt.

Mit den Merkmalen des Anspruchs 15 wird ein besonders einfacher und funktionsfähiger Anschluss eines Zeltdachrandes sowie des oberen Randes der Innenfolie in Verbindung mit einer Profilleiste beansprucht.

Anhand einer Zeichnung wird die Erfindung weiter erläutert.

### Es zeigen:

- Fig. 1: eine schematisierte teilweise geschnittene Seitenansicht einer Biogasanlage;
- Fig. 2: eine Draufsicht auf die Biogasanlage nach Fig. 1;
- Fig. 3: eine Anordnung der unteren Zugbänder eines Stützkäfigs;
- Fig. 4: einen Querschnitt durch einen Fermenterbehälter entlang der Linie B-B in Fig. 7;
- Fig. 5: einen Detailschnitt im Bereich von A aus Fig. 4;
- Fig. 6: eine Draufsicht auf eine Anordnung von oberen Aussteifungselementen des Stützkäfigs;
- Fig. 7: einen Längsschnitt durch einen fertig montierten Fermenterbehälter und
- Fig. 8: eine perspektivische Ansicht eines modifizierten Fermenterbehälters.

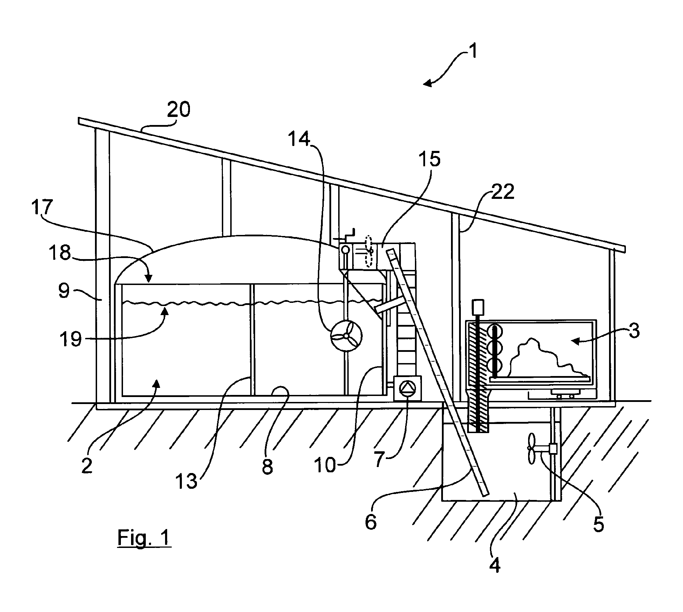

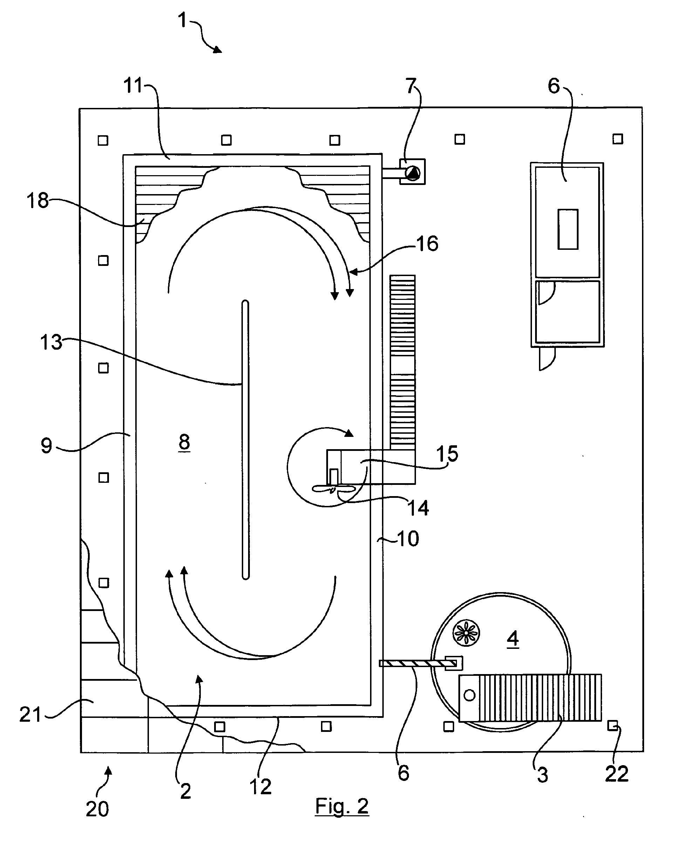

In den Fig. 1 und 2 ist schematisch eine Biogasanlage 1 gezeigt, wobei der spezielle Aufbau eines Fermenterbehälters 2 anhand der weiteren Figuren im Detail weiter unten erläutert wird.

Die Biogasanlage 1 besteht im Wesentlichen aus dem Fermenterbehälter 2, einem Feststoffbeschicker 3 mit Substrataufbereitung über einer Vorgrube 4 mit einem Rührwerk 5 und einer Beschickungsschnecke 6. Die Vorgrube 4 mit dem Feststoffbeschicker 3 ist neben dem Fermenterbehälter 1 angeordnet ebenso wie ein BHKW- und Technikcontainer 6 sowie eine Substratpumpe 7 zum Endlager und zum Rezirkulieren.

Der rechteckige Fermenterbehälter 2 weist eine Bodenwand 8, gegenüberliegende Längswände 9, 10 und Stirnwände 11, 12 auf. Zudem ist eine Strömungs-Längsmittenwand 13 angebracht, die beidseitig zu den Stirnwänden 11, 12 etwa mit einem Abstand entsprechend der Fermenterbehälterbreite beabstandet ist. Weiter ist ein höhenverstellbares und schwenkbares Tauchrührwerk 14 vorgesehen, welches zu Wartungszwecken an einem Servicepodest 15 aus dem Fermenterbehälter 2 herausgezogen werden kann. Mit dem Tauchrührwerk 14 wird um die Strömungslängsmittenwand 13 herum eine ringförmige Substratströmung (Pfeil 16) erzeugt. Der Fermenterbehälter 2 ist durch ein doppelwandiges Zeltdach 17 mit einem Gasspeicher abgedeckt, wobei an den Wandoberseiten ein Gurtnetz 18 mit längs- und/oder querverlaufenden Gurten aufgespannt ist. In Fig. 1 ist zudem die maximale Füllhöhe 19 mit ca. 3,7m bei einer Behälterhöhe von 4m eingezeichnet.

Die gesamte Biogasanlage 1 ist von einem Pultdach als Photovoltaikdach 20 mit Solarplattenelementen 21 überdacht. Das Photovoltaikdach 20 ist auf Stützen 22 angebracht.

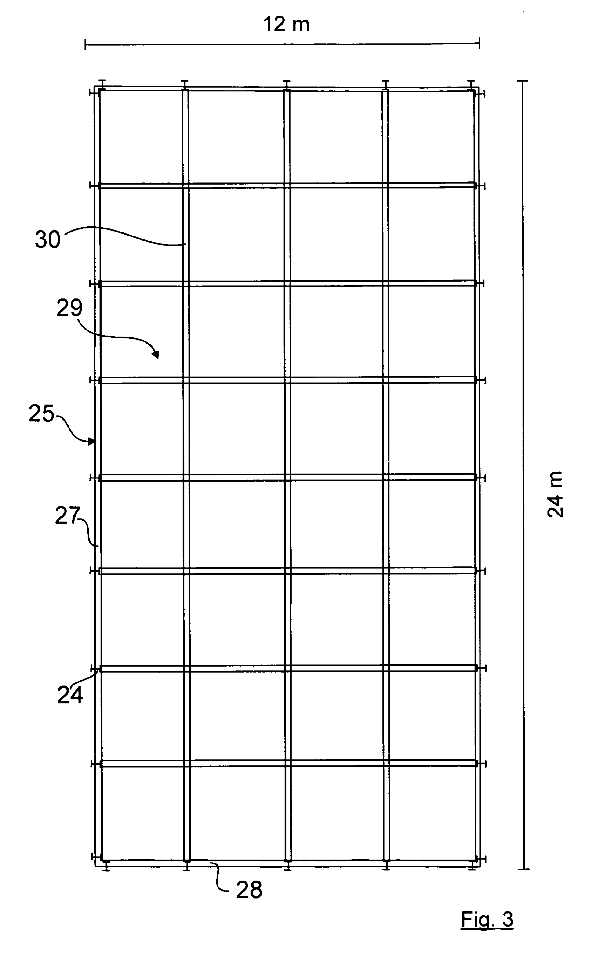

### In den Fig. 3 bis 6 ist der Aufbau des Fermenterbehälters 2 detailliert gezeigt:

Der Fermenterbehälter 2 weist einen quaderförmigen Stützkäfig 23 auf mit beabstandeten Vertikalstützen 24 als Stahl-Doppel-T-Träger sowie einem unteren Rechteckrahmen 25 und einem oberen Rechteckrahmen 26, die jeweils aus Längsträgern 27 und Querträgern 28 gebildet sind.

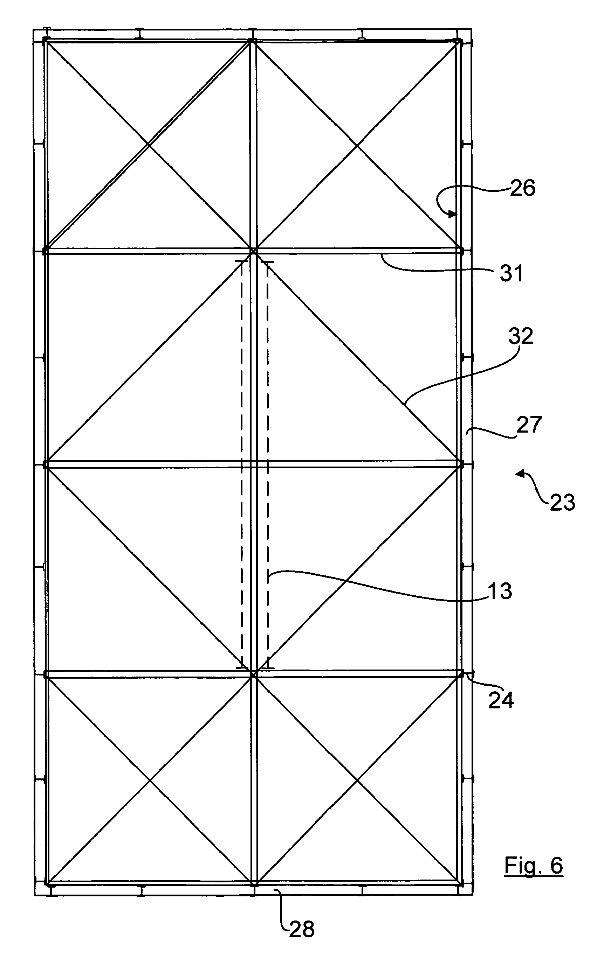

Im Bodenwandfeld des unteren Rechteckrahmens 25 (Fig. 3) ist ein Zugbandnetz 29 aus trägerparallelen Metallfachbändern 30 zwischen gegenüberliegenden Längsträgern 27 und Querträgern 28 angebracht. Im oberen Rechteckrahmen 26 (Fig. 6) sind Aussteifungsrohre 31 sowie sich überkreuzende Edelstahlseile 32 angebracht. Zudem ist hier strichlierte die Lage der Strömungslängsmittenwand 13 angedeutet.

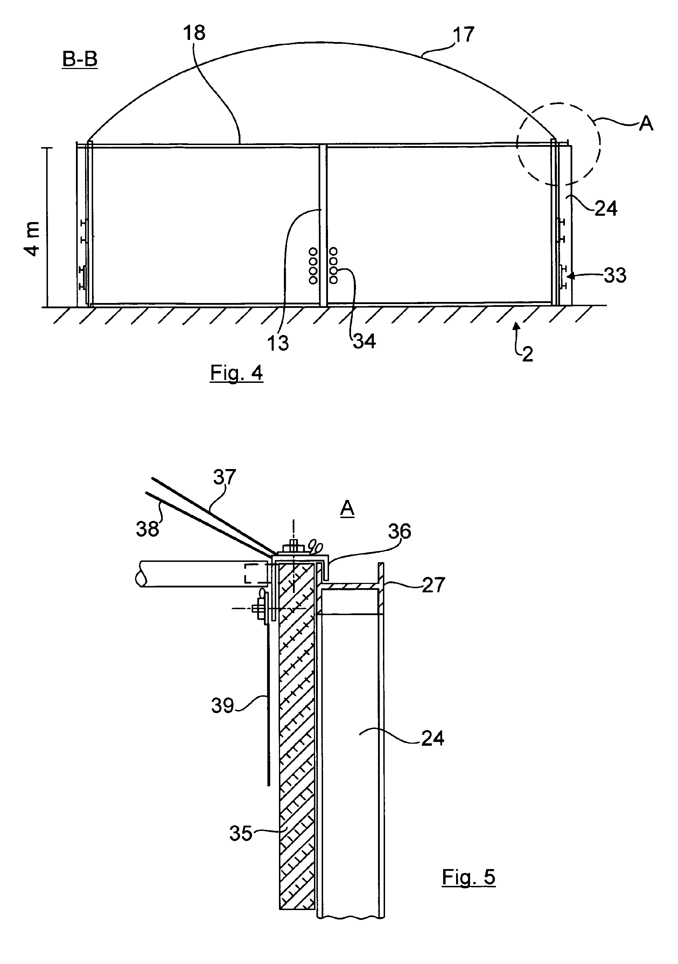

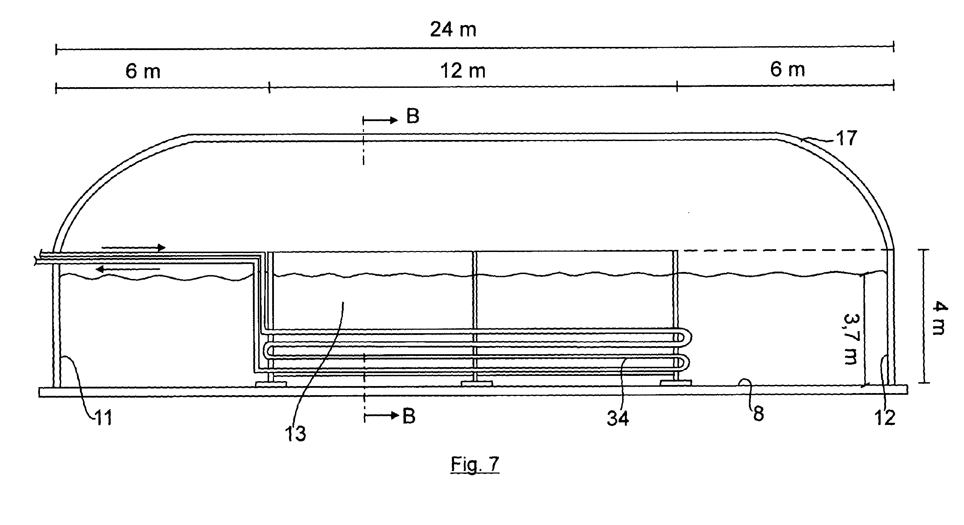

Im Querschnitt der Fig. 4 sind wiederum die Vertikalstützen 25 des Stützkäfigs 23 ersichtlich sowie weitere seitliche Aussteifungselemente 33 als weitere Längs- und Querträger. Zu beiden Seiten der Strömungs-Längsmittenwand 13 sind hier wie auch aus dem Längsschnitt der Fig. 7 ersichtlich im unteren Drittel verlaufenden Heizungsrohre 34 einer Fermenterheizung angebracht.

Aus dem Schnitt der Fig. 5 sind sowohl der Wandaufbau als auch Zeltdachanschluss im Detail ersichtlich. Der obere Längsträger 27 sowie eine die Beplankung bildende Sandwichpaneele 35 werden von oben her mit einer durchgehenden Profilleiste, hier eine U-Profilleiste 36 übergriffen, dabei sind die Ränder der zwei Dachmembranen 37, 38 an der oberen Profilbasis eingeklemmt und dicht befestigt. Der obere Rand einer Innenfolie 39, mit der der Fermenterbehälter 2 ausgekleidet ist, ist an einem fermenterinneren Profilschenkel der U-Profilleiste 36 dicht angeschlossen.

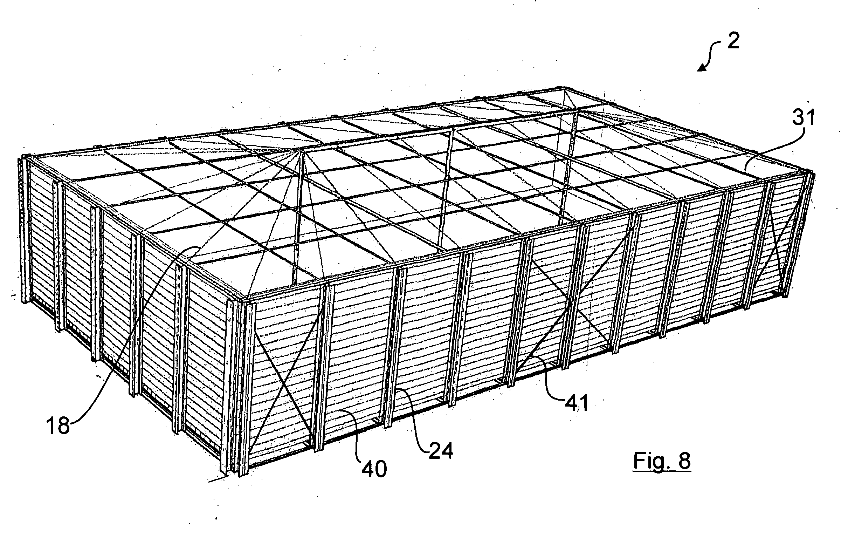

In Fig. 8 ist perspektivisch ein Fermenterbehälter 2 gezeigt mit Vertikalstützen 24 aus Stahl und einer dazwischenliegenden Beplankung aus horizontal verlaufenden Holzplattenelementen 40. Zudem sind auch hier Aussteifungsrohre 31 sowie ein Gurtnetz 18 ersichtlich. Zudem sind in einzelnen Seitenwandfeldern Kreuzverstrebungen 41 angebracht.

## Patentansprüche

1. Biogasanlage mit einem Fermenterbehälter, **dadurch gekennzeichnet,**
**dass** der Fermenterbehälter (2) mit einer rechteckigen Grundform eine Bodenwand (8), zwei gegenüberliegende Längswände (9, 10), zwei gegenüberliegende Stirnwände (11, 12) sowie eine obere Abdeckung (17) aufweist,
**dass** der Fermenterbehälter (2) zur Ausbildung seiner Wände aus einem quaderförmigen Stützkäfig (23) mit einer Gitterstruktur aus miteinander verbundenen, wandseitigen Vertikalstützen (24), Längsträgern (27), Querträgern (28) und gegebenenfalls Zugelementen (30, 32) und/oder Aussteifungselementen (31) besteht, wobei der Stützkäfig (23) zumindest zur Ausbildung der Längswände (9, 10) und Stirnwände (11, 12) mit Plattenelementen (39, 40) beplankt ist, und
**dass** die Bodenwand (8), die Längswände (9, 10) und die Stirnwände (11, 12) mit einer fermenterbehälterinneren, durchgehenden und dichten Innenfolie (39) ausgekleidet und abgedeckt sind.

2. Biogasanlage nach Anspruch 1, **dadurch gekennzeichnet, dass** die Gitterstrukturelemente des Stützkäfigs (23) und die Plattenelemente (39, 40) der Beplankung im nicht montierten Zustand einen Fermenterbehälter-Montagebausatz bilden, aus dem mit lösbaren Verbindungen Fermenterbehälter in gestuften standardisierten Größen herstellbar sind, vorzugsweise in einem Größenbereich von 24m Länge, 12m Breite und 4m Höhe für eine elektrische Leistung von ca. 150kW der Biogasanlage sowie in dagegen entsprechend abgestuften Größen für ca. 100kW, ca. 70kW und ca. 35kW, und
dass der Fermenterbehälter (2) sowie gegebenenfalls ein vorgefertigt zum Fermenterbehälter-Bausatz gehörender BHKW- und Technikcontainer (6) und gegebenenfalls weitere zur Biogasanlage (1) gehörende und neben dem Fermenterbehälter (2) angeordnete Vorrichtungen mit einem Pultdach als Photovoltaikdach (20) überdacht sind.

3. Biogasanlage nach Anspruch 1 oder 2, **dadurch gekennzeichnet,**
**dass** die Gitterstrukturelemente des Stützkäfigs (23) gebildet sind aus bodenseitigen unteren Längsträgern (27) und unteren Querträgern (28), aus denen ein unterer Rechteckrahmen (25) hergestellt ist,
aus Vertikalstützen (24) , die am unteren Rechteckrahmen (25) gegeneinander beabstandet, jeweils mit einem unteren Endbereich befestigt sind, und
aus oberen Längsträgern (27) und oberen Querträgern (28) , aus denen ein oberer Rechteckrahmen (26) hergestellt ist, der mit jeweils einem oberen Endbereich der Vertikalstützen (24) verbunden ist.

4. Biogasanlage nach Anspruch 3, **dadurch gekennzeichnet, dass** im Bodenwandfeld des unteren Rechteckrahmens ein Zugbandnetz (29) angebracht ist, welches mit Zugbändern, vorzugsweise mit trägerparallelen Metallflachbändern (30), jeweils gegenüberliegende Längsträger (27) und Querträger (28), bevorzugt im Bereich der Befestigungsstellen der Vertikalstützen (24) verbindet.

5. Biogasanlage nach Anspruch 3 oder 4, **dadurch gekennzeichnet,**
**dass** in der oberen Horizontalebene des oberen Rechteckrahmens (26) zwischen gegenüberliegenden Längsträgern (27) und/oder zwischen gegenüberliegenden Querträgern (28) trägerparallele Aussteifungselemente, vorzugsweise Aussteifungsrohre (31) und/oder schräg zu den Trägern verlaufende und sich überkreuzende Zugelemente, vorzugsweise Edelstahlseile (32) angebracht sind, und/oder
**dass** parallel und beabstandet zum unteren Rechteckrahmen (25) weitere Längsträger (33) und Querträger mit den Vertikalstützen (24) verbunden sind, die vorzugsweise wieder einen geschlossenen Rechteckrahmen bilden, und/oder
**dass** in den Seitenwandfeldern zwischen den Vertikalstützen (24) in jeweils einem Seitenwandfeld oder zwei oder mehrere Seitenwandfelder übergreifend und sich gegebenenfalls kreuzende Aussteifungselemente und Zugelemente, vorzugsweise Metallstäbe (41) angebracht sind.

6. Biogasanlage nach einem der Ansprüche 1 bis 5, **dadurch gekennzeichnet, dass** die Vertikalstützen (24), die Längsträger (27), die Querträger (28) und gegebenenfalls Zugelemente und Aussteifungselemente aus Metall, vorzugsweise die Vertikalstützen (24) und Längsträger (27) als Stahl-Doppel-T-Träger und/oder StahlWinkelträger, oder aus Holz hergestellt und miteinander verschraubt sind.

7. Biogasanlage nach einem der Ansprüche 1 bis 6, **dadurch gekennzeichnet, dass** die Bodenwand des Fermenterbehälters (2) durch einen planbefestigten Erdboden oder eine unbewehrte Magerbetonplatte gebildet ist, auf der eine Wärmedämmungsschicht aus Wärmedämmungsplatten und darüber die Innenfolie aufliegt, wobei gegebenenfalls ein bodenseitiges Zugbandnetz (29) abgedeckt, insbesondere eingesandet oder im Magerbeton eingebettet ist und auf dieser Abdeckschicht die Wärmedämmungsschicht und die Innenfolie (39) aufliegt.

8. Biogasanlage nach einem der Ansprüche 1 bis 7, **dadurch gekennzeichnet, dass** die Plattenelemente als Beplankung der Längswände und Stirnwände wasserfeste Sandwichpaneele (35) mit integriertem Dämmmaterialkern und/oder Stahlplatten mit aufgebrachter Wärmedämmschicht und Verkleidung und/oder Kunststoffplatten oder Holzfaserplatten (40) oder Holzbretter mit aufgebrachter Wärmedämmschicht und Verkleidung sind.

9. Biogasanlage nach Anspruch 8, **dadurch gekennzeichnet, dass** die Plattenelemente (35, 40) Rechteckplatten sind mit einer Länge entsprechend der Fermenterbehälterhöhe und vertikal gerichtet von innen her an die Stützkäfigseitenwänden anliegen oder mit einer Länge entsprechend der Breite eines Seitenwandfeldes horizontal gerichtet zwischen Vertikalstützen (24) verbaut sind.

10. Biogasanlage nach einem der Ansprüche 1 bis 9, **dadurch gekennzeichnet, dass** der Fermenterbehälter (2) eine jeweils zu den Stirnwänden (11, 12) beabstandete Strömungs-Längsmittenwand (13) aufweist, die vorzugsweise bei einem Fermenterbehälter-Montagebausatz als Montagemodul vorgefertigt ist,
dass wenigstens ein Rührwerk als Tauchrührwerk (14) oder als Stabrührwerk zum Aufbau einer ringförmigen Substratströmung um die Strömungs-Längsmittenwand (13) herum vorgesehen ist.

11. Biogasanlage nach Anspruch 10, **dadurch gekennzeichnet, dass** auf der Strömungs-Längsmittenwand (13) und/oder in dieser integriert Heizelemente (34) für die Fermenterheizung angebracht sind.

12. Biogasanlage nach Anspruch 11, **dadurch gekennzeichnet, dass** die Heizelemente beidseitig an der Strömungs-Längsmittenwand (13), vorzugsweise parallel im unteren Drittel verlaufende Heizungsrohre (34) sind und die Strömungs-Längsmittenwand (13) aus festem Material und/oder aus einem an einem Gestell befestigten flexiblen Gewebe besteht.

13. Biogasanlage nach einem der Ansprüche 1 bis 12, **dadurch gekennzeichnet, dass** die Innenfolie (39) als gewebeverstärkte PVC-Folie und/oder als FPP-Dichtungsbahn ausgeführt ist, und dass weitere Bauelemente, insbesondere dem Fermenterinnenbereich ausgesetzte Bauelemente, aus V4A-Stahl hergestellt oder mit Epoxidharz beschichtet sind.

14. Biogasanlage nach einem der Ansprüche 1 bis 13, **dadurch gekennzeichnet, dass** als obere Abdeckung des Fermenterbehälters (2) ein Zeltdach (17), vorzugsweise ein doppelwandiges Zeltdach aus zwei Dachmembranen (37, 38) mit integriertem Gasspeicher, sowie einem in der Höhe der Längswände und Stirnwände bei entsprechender Höhe der Strömungs-Längsmittenwand (13) aufgespannten, längs und/oder quer verlaufenden Gurtnetz (18) vorgesehen ist.

15. Biogasanlage nach Anspruch 14, **dadurch gekennzeichnet, dass** der obere Rand der Längswände (9, 10) und Stirnwände (11, 12) als Wandkrone, insbesondere die dortigen Plattenelementränder der Beplankung von einer durchgehenden Profilleiste (36) übergriffen werden, an deren oberer Profilbasis der Zeltdachrand, vorzugsweise die Ränder der zwei Dachmembranen (37, 38) dicht befestigt sind und an einem fermenterinneren Profil-Schenkel entsprechend der obere Rand der Innenfolie (39) dicht angeschlossen ist.
